# EUROPÄISCHE PATENTSCHRIFT

(11) **EP 3 113 806 B1**
(45) Veröffentlichungstag und Bekanntmachung des Hinweises auf die Patenterteilung: **31.03.2021**
(21) Anmeldenummer: 15715969.0
(22) Anmeldetag: 02.03.2015
(51) Int. Cl.: A61M 1/10, A61M 1/12, A61M 1/36

(54) **KATHETER ZUM GERICHTETEN LEITEN EINER KÖRPERFLÜSSIGKEIT**
CATHETER FOR CONDUCTING A BODILY FLUID IN A DIRECTED MANNER
CATHÉTER POUR L'ÉCOULEMENT DIRIGÉ D'UN LIQUIDE CORPOREL

(30) Priorität: 03.03.2014 DE 102014003153
(43) Veröffentlichungstag der Anmeldung: 11.01.2017
(73) Patentinhaber: Novapump GmbH, 07749 Jena (DE)
(72) Erfinder: PFEIFER, Jörg, 07743 Jena (DE); REICH, Ronald, 07749 Jena (DE)
(74) Vertreter: Carlsohn, Marc René
(86) Internationale Anmeldenummer: PCT/DE2015/100081
(87) Internationale Veröffentlichungsnummer: WO 2015/131879

(56) Entgegenhaltungen:
- EP-A1- 1 066 066
- EP-A2- 0 608 846
- WO-A1-97/02850
- GB-A- 1 370 546
- US-A1- 2009 270 815

## Beschreibung

Die Erfindung betrifft einen Katheter zum gerichteten Leiten eines (Körper-) Fluids, insbesondere einer Körperflüssigkeit, umfassend eine Hülle mit einem Innenraum und einen Rahmen, wobei die Hülle mindestens drei Öffnungen aufweist (und abseits dieser Öffnungen gegenüber dem zu leitenden Fluid fluiddicht ist), und zumindest in einem Bereich zwischen erster Öffnung und zweiter Öffnung als Leitung für das Fluid ausgebildet ist, und wobei an der zweiten Öffnung ein Rückschlagventil (zum Ermöglichen einer ausschließlich unidirektionalen Strömung zwischen dem Innenvolumen der Hülle und dem die Hülle umgebenden Außenraum) angeordnet ist.

Die Eigenschaft "fluiddicht" bedeutet im Sinne der Erfindung eine Undurchlässigkeit gegenüber Atomen oder Molekülen des betreffenden Fluids unter den im lebenden menschlichen Körper in diesem Fluid maximal herrschenden Bedingungen. Ist der Katheter beispielsweise zum Leiten von Blut ausgebildet, so bedeutet "fluiddicht" eine Undurchlässigkeit mindestens bis zu einem maximalen menschlichen Blutdruck.

Ein Katheter der eingangs genannten Art mit mehreren Rückschlagventilen ist als Stand der Technik beispielsweise aus WO 2008/113785 A2 oder EP 1 066 066 A1 bekannt. Er wird vorzugsweise bei eingeschränkter Herzleistung zur Unterstützung des Herzens und des Blutkreislaufes eingesetzt. Insbesondere kann er auch bei einer höhergradigen Aortenklappeninsuffizienz eingesetzt werden. Er dient dazu, das zu leitende Fluid von einem ersten Ort an einen anderen Ort zu transportieren, ohne den Druck des Fluides am ersten Ort signifikant über den physiologisch vorgegeben Zustand hinaus zu erhöhen, indem er das Prinzip einer Tauchpumpe realisiert und vorzugsweise durch Einsatz eines Ballonkatheters mit dem Prinzip einer Membranpumpe kombiniert. Die Leitrichtung (Strömungsrichtung) hängt dabei von der Orientierung der Rückschlagventile ab. Er ermöglicht so gegenüber der bekannten intraaortalen Ballongegenpulsation einen gerichteten Transport des Körperfluides bei zudem geringerer Belastung des Patienten.

Derartige Katheter können vereinfacht als Pumpkatheter bezeichnet werden. Es ist jedoch möglich, einen separaten Antrieb, insbesondere in Form eines einstellbaren Verdrängers, beispielsweise einen Ballonkatheter einer intraaortalen Ballonpumpe (IABP), zu nutzen. Der Katheter ist dann in seiner Grundform lediglich ein antriebsloser Leitungskatheter. Der Pumpkatheter kann dann bereitgestellt werden, indem der Verdränger nach dem Platzieren des Leitungskatheters durch die dritte Öffnung in den Innenraum der Hülle geschoben wird.

Eine Bereitstellung des Leitungskatheters ist daher auch ohne Antrieb sinnvoll möglich.

WO 97/02850A1 offenbart einen Pumpkatheter mit einer extrakorporal angeordneten Pumpkammer sowie verschiedene Ventilausführungen für diesen Katheter. US 2009/2708215 A1 zeigt Katheter, die verschiedene Arten von Auslassventilen aufweisen. EP 0 608 846 A2 zeigt Vorrichtungen zur Wunddrainage mit verschiedenen Einlassventilen. Der Aufwand und die Beeinträchtigung des Patienten beim minimalinvasiven Einführen eines Katheters in den Körper, beispielsweise über Leistengefäße, hängen wesentlich von der Größe, insbesondere dem größten Außendurchmesser, des Katheters ab.

Der Erfindung liegt daher die Aufgabe zugrunde, einen Katheter der eingangs genannten Art anzugeben, der eine geringere Größe aufweist.

Diese Aufgabe wird durch einen Katheter gelöst, welcher die in Anspruch 1 angegebenen Merkmale aufweist. In den Unteransprüchen sind vorteilhafte Ausgestaltungen angegeben.

Die Erfindung sieht vor, dass das Rückschlagventil eine (flexible) Ventilfolie umfasst, die an der Hülle zumindest teilweise befestigt ist, so dass die zweite Öffnung durch die Ventilfolie (reversibel) vollständig bedeckbar ist. Im Sinne der Erfindung ist die Ventilfolie ein Blatt beliebigen Materials mit einer Dicke von maximal 0,2 mm. Reversibel bedeutet, dass die zweite Öffnung durch die Ventilfolie alternierend freigebbar und bedeckbar ist.

Die Ventilfolie bildet mit der Hülle somit ein Folienventil, das von dem Fluid nur unidirektional durchströmbar ist. In Durchlassrichtung kann gegen die Ventilfolie drückendes Fluid die Ventilfolie von der zweiten Öffnung abheben und so das Folienventil passieren. In Sperrrichtung drückt das Fluid die Ventilfolie gegen die zweite Öffnung, die dadurch bedeckt wird. Das Fluid kann das Folienventil dann nicht passieren. Gegenüber dem Stand der Technik, der mechanisch steife und damit bauraumintensive Rückschlagventile beschreibt, trägt das erfindungsgemäß vorgesehene Folienventil aufgrund der geringen Dicke der Ventilfolie nur in vernachlässigbarem Umfang auf, so dass der Katheter im gefalteten Zustand einen gegenüber dem Stand der Technik geringeren größten Außendurchmesser (nachfolgend auch als Gesamtdurchmesser bezeichnet) aufweisen kann. Das ist insbesondere innerhalb der Kardiologie vorteilhaft, da erfahrungsgemäß bei verringerten Gesamtdurchmesser von Herzkathetern weniger Patienten eines bestimmten Patientenkollektivs aufgrund ihres individuellen Gefäßinnendurchmessers von einem Eingriff ausgeschlossen werden müssen. Zudem kann die Sicherheit der Implantation und Explantation durch den geringeren Gesamtdurchmesser verbessert werden.

Besteht keine Druckdifferenz zwischen Innenraum und Außenraum, so kann die Ventilfolie entweder aufgrund einer mechanischen Vorspannung und/oder aufgrund ihres geometrischen Verlaufs an der Hülle anliegen oder sich lose in einer ausschließlich durch die Befestigung an der Hülle definierten Lage relativ zu der Hülle befinden.

Allgemein kann die Hülle, insbesondere im Bereich einer radiärsymmetrischen beziehungsweise rotationssymmetrischen Mantelfläche, so geformt sein, dass die zweite Öffnung einen nicht-ebenen, beispielsweise in drei Dimensionen gekrümmten Rand aufweist, beispielsweise im Falle einer zylindrischen Mantelfläche. Nur die erfindungsgemäßen Folienventile erlauben bei geringem Konstruktionsaufwand den fluiddichten Verschluss von Öffnungen mit dreidimensional gekrümmten Rändern.

Die Ventilfolie kann unmittelbar oder mittelbar an der Hülle befestigt sein. Zur unmittelbaren Befestigung kann die Ventilfolie beispielsweise entlang einer Befestigungslinie an die Hülle geklebt, geschweißt oder geklemmt sein. Die Befestigung kann entlang der Befestigungslinie durchgehend oder unterbrochen, beispielsweise punktförmig, sein. Zur mittelbaren Befestigung kann die Ventilfolie beispielsweise entlang der Befestigungslinie an einem Zwischenkörper befestigt sein, sei es durch Kleben, Schweißen oder Klemmen, wobei der Zwischenkörper seinerseits beispielsweise entlang einer Hilfsbefestigungslinie mittelbar oder unmittelbar an der Hülle beziehungsweise einem weiteren Zwischenkörper befestigt sein kann. Der Zwischenkörper kann beispielsweise durch den Rahmen gebildet sein.

Die Befestigungslinie kann eine beliebige Form aufweisen. Beispielsweise kann sie innerhalb der Ebene der Ventilfolie gerade sein. Es kann insbesondere vorgesehen sein, dass die Befestigungslinie im wesentlichen eine Gerade bildet oder die zweite Öffnung teilweise umschließt.

Bei einer positiven Druckdifferenz in Sperrrichtung des Rückschlagventils liegt die Ventilfolie im wesentlichen (also abgesehen von Falten) flächig an der Hülle an und verdeckt auf diese Weise die zweite Öffnung fluiddicht. Bei einer negativen Druckdifferenz wird die Ventilfolie dagegen in den Bereichen abseits der Befestigungslinie von der Hülle weggedrückt, so dass Fluid das Rückschlagventil in Durchlassrichtung passieren kann.

Vorzugsweise weist die Ventilfolie mindestens ein Loch auf, das im vollständig bedeckten Zustand der zweiten Öffnung abseits der zweiten Öffnung angeordnet ist. Das Loch ist also gegenüber der zweiten Öffnung so versetzt, dass Loch und zweite Öffnung im bedeckten Zustand überlappungsfrei sind. Die Ventilfolie liegt dann bei einer positiven Druckdifferenz in Sperrrichtung des Rückschlagventils im wesentlichen (also abgesehen von Falten) flächig an der Hülle an und ist im wesentlichen parallel zu deren Oberfläche. Auf diese Weise verdeckt sie die zweite Öffnung fluiddicht.

Insbesondere kann die Befestigungslinie derart verlaufen, dass die zweite Öffnung und das Loch gemeinsam von der Linie umschlossen sind. Durch die so verringerte Beweglichkeit wird eine höhere Betriebssicherheit bei hoher Dichtigkeit erreicht. Insbesondere kann die Ventilfolie durch die um die zweite Öffnung allseitige Befestigung an der Hülle von dem Körperfluid nur um eine definierte maximale Tiefe in die zweite Öffnung hineingezwungen werden. Die Tiefe hängt einerseits von der relativen Größe der durch die Befestigungslinie umgrenzten Fläche der Ventilfolie gegenüber der durch die Befestigungslinie (beziehungsweise, sofern vorhanden, die Hilfsbefestigungslinie) umgrenzten Fläche der Hülle und andererseits von der Elastizität der Ventilfolie und der Hülle ab. Sofern die Ventilfolie und/oder die Hülle durch die Befestigung mit einer jeweiligen inneren Zugspannung beaufschlagt ist, hängt die maximale Tiefe auch von dieser ab. Vorteilhaft ist es, wenn das Loch der Ventilfolie eine Fläche zwischen 5 mm² und 500 mm² aufweist. Besonders vorteilhaft weist das Loch eine Fläche zwischen 10 mm² und 200 mm² auf. Es ist auch möglich, dass die Ventilfolie mehrere Löcher aufweist, die beim Bedecken der zweiten Öffnung alle abseits der zweiten Öffnung liegen. In diesem Fall weisen diese Löcher der Ventilfolie zusammen vorzugsweise eine Gesamtfläche zwischen 5 mm² und 500 mm² und besonders bevorzugt zwischen 10 mm² und 200 mm² auf (wobei ein einzelnes Loch der mehreren Löcher bevorzugt eine Fläche zwischen 0,25 mm² und 250 mm² und besonders bevorzugt zwischen 1 mm² und 20 mm² aufweist). Diese (Gesamt-) Lochfläche ermöglicht einerseits eine hohe Dichtigkeit des Rückschlagventils, andererseits einen geringen Strömungswiderstand gegenüber dem zu leitenden Fluid. Die Befestigungslinie kann dann beispielsweise rings um alle Löcher gemeinsam verlaufen.

In einer alternativen Ausführungsform kann das Rückschlagventil eine Zusatzfolie umfassen, die teilweise flächig an der Ventilfolie anliegt und (fluiddicht) an der Hülle befestigt ist, wobei die Befestigungslinie der Ventilfolie zusammen mit der Befestigungslinie der Zusatzfolie die zweite Öffnung ringsum umgibt. Die zweite Öffnung ist dann von den Befestigungslinien der Ventilfolie und der Zusatzfolie umschlossen. Ein solches Folienventil kann als Auslassventil beispielsweise gemäß DE 35 25 165 A1 ausgeführt sein.

Vorzugsweise ist die Hülle zumindest abschnittsweise, insbesondere abseits ihrer Enden, oder zumindest ihrer äußeren Einhüllenden exakt oder im wesentlichen radiärsymmetrisch oder rotationssymmetrisch (unendlich-zählig radiärsymmetrisch) um eine Längsachse, insbesondere zylinderförmig, und die zweite Öffnung in einer (die Längsachse umgebenden) Mantelfläche, insbesondere einer Zylindermantelfläche, der Hülle angeordnet. Der kleinste Außendurchmesser entspricht dann dem Querschnitt zur Längsachse. Je höherzähliger die Radiärsymmetrie ist, umso geringer kann vorteilhafterweise der kleinste Außendurchmesser sein. Es ist zweckmäßig, die dritte Öffnung auf der Längsachse anzuordnen.

Die Hülle umfasst (neben dem Leitungsabschnitt) vorzugsweise einen Pumpkammerabschnitt. Zwischen dem Leitungsabschnitt und dem Pumpkammerabschnitt können ein oder mehrere Verbindungsabschnitte angeordnet sein. Der Pumpkammerabschnitt kann ganz oder teilweise ein Unterabschnitt des Leitungsabschnittes sein. Zweckmäßigerweise ist die erste Öffnung der Hülle im Bereich eines Endes des Leitungsabschnitts angeordnet und die zweite Öffnung im Bereich eines entgegengesetzten Endes des Leitungsabschnitts angeordnet.

In einer bevorzugten Ausführungsform ist die zweite Öffnung im Bereich des Pumpkammerabschnittes der Hülle angeordnet. In diesem Fall bildet der Pumpkammerabschnitt einen Teil des Leitungsabschnitts, so dass die Pumpenkammer Teil der Leitung ist.

In einer besonderes für die Anwendung am linken Herzen geeigneten Ausführungsform kann die zweite Öffnung jedoch auch abseits des Pumpkammerabschnitts in dem Leitungsabschnitt angeordnet sein.

Typischerweise weist die Hülle im Bereich ihres Pumpkammerabschnitts einen größeren Innendurchmesser auf als im Bereich des Leitungsabschnitts.

Die Hülle kann einteilig oder mehrteilig ausgebildet sein. Bei einer Ausführung aus mehreren Teilen kann die Hülle zweckmäßigerweise aus einem abseits des Rahmens angeordneten Teil, das den Leitungsabschnitt bereitstellt, und einem im Bereich des Rahmens angeordneten Teil, das den Pumpkammerabschnitt bereitstellt, ausgebildet sein. Bei einer Ausführung der Hülle aus mehreren Teilen können diese aus unterschiedlichen Materialien bestehen und zweckmäßigerweise fluiddicht miteinander verbunden sein. Die Hülle oder deren Teile können auch jeweils für sich aus unterschiedlichen Materialien bestehen. Insbesondere können sie mindestens zweischichtig aus Schichten unterschiedlichen Materials sein. Beispielsweise kann eine Trägerschicht, die der Hülle oder dem Teil eine vorbestimmte mechanische Steifigkeit verleiht, und eine mit der Trägerschicht verbundene Hüllschicht zur fluiddichten Ausbildung der Hülle vorgesehen sein. Sofern der Rahmen durch die Hülle selbst gebildet ist, kann die Trägerschicht durch den Rahmen gebildet sein.

Vorzugsweise ist die Hülle zumindest in dem Pumpkammerabschnitt durch mindestens eine Hüllfolie gebildet. Die Hüllfolie kann ihrerseits mehrschichtig sein. Bevorzugt ist die Hüllfolie zumindest stellenweise (mittelbar oder unmittelbar, s. o.) mit dem Rahmen verbunden. Durch ihre geringe Dicke ist so auch die Hülle bauraumminimiert. Das ermöglicht einen noch geringeren Außendurchmesser des Katheters (in der zusammengefalteten Konfiguration des Rahmens) im Bereich der Pumpenkammer. Die Hüllfolie kann beispielsweise wie in einem Gewebe durch die Streben des Rahmens abwechselnd zwischen dem Innenraum und dem Außenraum hin- und hergeführt sein.

Zweckmäßigerweise ist dabei die Hüllfolie (bezüglich des Rahmens) weniger weit auslenkbar als (bei identischer Auslenkkraft) die Ventilfolie und/oder durch den Rahmen mit einer größeren inneren Zugspannung beaufschlagt als die Ventilfolie, insbesondere mit einer kleineren Dehnbarkeit der Hüllfolie als die der Ventilfolie. Auf diese Weise kann einerseits eine hohe Dichtigkeit des Rückschlagventils, andererseits ein geringer Strömungswiderstand gegenüber dem zu leitenden Fluid erreicht werden.

In allen Ausführungsformen kann die Ventilfolie (und gegebenenfalls die Zusatzfolie) vorzugsweise innerhalb der Hülle angeordnet sein, so dass das Rückschlagventil als Einlassventil wirkt. Diese Ausgestaltung ist besonders für den Einsatz im Blutkreislauf am rechten Herzen vorteilhaft.

Alternativ kann die Ventilfolie in allen Ausführungsformen außerhalb der Hülle angeordnet sein, so dass das Rückschlagventil als Auslassventil wirkt. Auch damit werden die Vorteile der Erfindung erzielt.

In einer vorteilhaften Ausgestaltungsvariante ist die dritte Öffnung der Hülle so ausgebildet, dass ein Antrieb, insbesondere ein Ballon eines Ballonkatheters, insbesondere einer IABP, durch die dritte Öffnung in den Innenraum der Hülle bis zu einer vorbestimmten Zielposition relativ zu der Hülle schiebbar ist. Bevorzugt entspricht die vorbestimmte Zielposition dem Bereich des Pumpkammerabschnittes der Hülle. Zweckmäßigerweise ist der Antrieb so durch die dritte Öffnung schiebbar, dass diese (fluiddicht, insbesondere zumindest gegenüber einen maximalen Blutdruck) verschlossen ist. Als Antrieb in Form eines Verdrängers kann beispielsweise ein Katheter gemäß US 5,460,607 A eingesetzt werden. Der im Innenraum angeordnete Antrieb kann über eine durch die dritte Öffnung hindurchführende Leitung mit einer externen Energiequelle verbunden sein, im Falle eines Ballonkatheters beispielsweise über eine Hilfsfluidleitung mit einer Pumpkonsole, die den Ballon mit einem Hilfsfluid vorzugsweise stoßartig befüllen und entleeren kann. Aufgrund der verdrängenden Wirkung des befüllten Ballons wirkt die Pumpkonsole als Antrieb für das erfindungsgemäße Folien-Rückschlagventil und ermöglicht damit einen gerichteten Transport der Körperflüssigkeit. Der Antrieb kann beispielsweise hinsichtlich der Frequenz der Befüllvorgänge des Ballons mit Hilfsfluid und/oder des Volumens an Hilfsfluid pro Befüllvorgang einstellbar sein. Erfindungsgemäß können auch alternative Ausführungen des Antriebs verwendet werden, beispielsweise Antriebe nach dem Prinzip einer Kolbenpumpe oder einer Impellerpumpe oder auch Antriebe nach dem Prinzip einer Kreiselpumpe.

In einer besonders vorteilhaften Ausgestaltungsvariante ist im Innenvolumen der Hülle ein Ballon (eines Ballonkatheters, insbesondere einer IABP) angeordnet und an den Ballon eine Leitung für ein Hilfsfluid zum (reversiblen) Aufpumpen des Ballons angeschlossen, wobei die Leitung für das Hilfsfluid durch die dritte Öffnung der Hülle nach außen verläuft. Die dritte Öffnung ist dann durch die Leitung für das Hilfsfluid fluiddicht verschlossen. Dadurch ist der Katheter ohne die zusätzlichen Arbeitsschritte des nachträglichen Einbringens eines separaten Verdrängers in das zu leitende Fluid und des Einschiebens des Ballons in den Innenraum des Leitungskatheters einsatzbereit. Die Behandlungsdauer wird dadurch verringert. Insbesondere kann die Leitung für das Hilfsfluid mit einer Pumpe (Pumpkonsole) für das Hilfsfluid verbindbar oder verbunden sein.

Der Rahmen ist im wesentlichen schlauchförmig ausgebildet. Vorzugsweise ist der Rahmen zumindest abschnittsweise, insbesondere abseits seiner Enden, oder zumindest seiner äußeren Einhüllenden exakt oder im wesentlichen radiärsymmetrisch oder rotationssymmetrisch (unendlich-zählig radiärsymmetrisch) um eine Längsachse, insbesondere zylinderförmig angeordnet. Bei dem erfindungsgemäßen Katheter ist der Rahmen bevorzugt zumindest teilweise entlang des Bereiches des Pumpkammerabschnittes der Hülle angeordnet. Der Rahmen kann bevorzugt aufstellbar sein. Die Eigenschaft "aufstellbar" bedeutet im Sinne der Erfindung, dass der Rahmen zwischen zwei Konfigurationen mit unterschiedlichen Innenvolumen umschaltbar ist. Das Umschalten in die Konfiguration mit größerem Innenvolumen kann als "Aufstellen" bezeichnet werden, die andere Konfiguration kann als "gefaltet" bezeichnet werden. Insbesondere kann der Rahmen somit ein aufstellbarer Stent sein. Der Rahmen kann im Innenraum der Hülle oder außen um die Hülle herum angeordnet sein. Der Rahmen kann aber auch durch die Hülle selbst gebildet sein.

Es kann vorteilhaft sein, wenn der Rahmen zumindest einen Teil der Hülle, insbesondere den Pumpkammerabschnitt, und die dritte Öffnung, insbesondere auch die zweite Öffnung, (starr) bildet. Dadurch ist kein zusätzliches, auftragendes Material für die Hülle notwendig.

Der Rahmen kann zweckmäßigerweise eine Zusammensetzung aufweisen, die eine Formgedächtnislegierung, insbesondere Nitinol, ein Formgedächtnispolymer oder eine Formgedächtniskeramik umfasst oder aus einer solchen besteht. Insbesondere kann die Umstellbarkeit zwischen den Konfigurationen reversibel sein.

In seiner funktionalen Konfiguration mit größerem Innenvolumen spannt der Rahmen die Hülle im Bereich ihres Pumpkammerabschnittes zu einer Pumpenkammer auf. Das Fluid kann entlang der von dem Leitungsabschnitt bereitgestellten Leitung entweder von der ersten Öffnung zur zweiten Öffnung oder umgekehrt durch die Hülle transportiert werden. Die Transportrichtung hängt von der Orientierung des an der zweiten Öffnung angeordneten Rückschlagventils ab.

Vorteilhafterweise kann das Rückschlagventil eine Gruppe mehrerer zweiter Öffnungen umfassen, wobei von der Ventilfolie diese Öffnungen der betreffenden Gruppe vollständig bedeckbar sind. Auf diese Weise können die Dichtheit gesteigert und der Strömungswiderstand gesenkt werden. Die Befestigungslinie kann dann rings um alle zur Gruppe gehörigen zweiten Löcher gemeinsam verlaufen.

Besonders bevorzugt sind Ausführungsformen, bei denen vorzugsweise in der Mantelfläche der im wesentlichen zylindrischen Hülle mehrere zweite Öffnungen mit einem jeweiligen Rückschlagventil angeordnet sind und jeweils eine Ventilfolie oder ein jeweiliges durch Befestigungslinien definiertes Segment der Ventilfolie mit einem jeweiligen Loch oder einer jeweiligen (zu dem betreffenden Rückschlagventil gehörigen) Gruppe von Löchern zum Bedecken der betreffenden zweiten Öffnung angeordnet ist. Auf diese Weise kann der Strömungswiderstand des Leitungskatheters bei gesteigerter Dichtheit reduziert werden.

Ebenso vorteilhaft kann jedes Rückschlagventil eine Gruppe mehrerer zweiter Öffnungen umfassen, wobei von einer jeweiligen Ventilfolie oder einem (durch eine jeweilige Befestigungslinie definierten) Segment der Ventilfolie die Öffnungen der betreffenden Gruppe zweiter Öffnungen vollständig bedeckbar sind. Auf diese Weise kann der Strömungswiderstand des Leitungskatheters bei gesteigerter Dichtheit reduziert werden. Die zu einer Gruppe gehörige Befestigungslinie kann beispielsweise dann rings um alle zur betreffenden Gruppe gehörigen zweiten Öffnungen gemeinsam verlaufen.

Vorzugsweise ist in der Leitung für das Körperfluid oder am Ende dieser Leitung ein weiteres Ventil, insbesondere ein Rückschlagventil, angeordnet, das entgegengesetzt zu dem mindestens einen ersten Ventil wirkt. Dadurch wird die Effizienz des gerichteten Transports verbessert.

Vorzugsweise ist die Leitung flexibel, insbesondere handelt es sich einen flexiblen Schlauch. Die Leitung für das Körperfluid weist an ihrem von dem Rahmen entfernten Ende eine elastische Spirale auf. Dadurch kann das Leitungsende im Körper an einer vorbestimmten Position gehalten und insbesondere im Blutgefäß von der Gefäßwandung allseits beabstandet fixiert werden.

Vorzugsweise weist jede zweite Öffnung eine Fläche zwischen 5 mm² und 500 mm² auf. Besonders bevorzugt weist jede zweite Öffnung eine Fläche zwischen 10 mm² und 200 mm² auf. Für den Fall, dass das Rückschlagventil eine Gruppe von zweiten Öffnungen umfasst, weist jede zu einem Rückschlagventil gehörige Gruppe von zweiten Öffnungen bevorzugt eine Gesamtfläche zwischen 5 mm² und 500 mm² und besonders bevorzugt zwischen 10 mm² und 200 mm² auf (wobei eine einzelne Öffnung der Gruppe von zweiten Öffnungen vorteilhaft eine Fläche von jeweils zwischen 0,25 mm² und 250 mm² und besonders vorteilhaft zwischen 1 mm² und 20 mm² aufweist). Diese (Gesamt-) Öffnungsfläche ermöglicht einerseits eine hohe Dichtigkeit des Rückschlagventils, andererseits einen geringen Strömungswiderstand gegenüber dem zu leitenden Fluid.

Zweckmäßigerweise können die erste Folie und/oder die zweite Folie aus mindestens einem Polymer, insbesondere Polyurethan, bestehen, insbesondere mit einer Foliendicke zwischen 0,01 mm und 0,2 mm. Das ermöglicht eine bauraumminimale erste Konfiguration des Katheters.

Nachfolgend wird die Erfindung anhand von Zeichnungen näher erläutert. Darin zeigt
Fig. 1 einen bekannten Leitungskatheter mit herkömmlichen Rückschlagventilen gemäß dem Stand der Technik,
Fig. 2 einen Leitungskatheter mit mehreren Einlass-Rückschlagventilen in Form von Folienventilen mit separaten ersten Folien und integriertem Pumpballon in zwei verschiedenen Zuständen,
Fig. 3 einen Leitungskatheter mit mehreren Einlass-Rückschlagventilen in Form von Folienventilen mit einer gemeinsamen ersten Folie,
Fig. 4 einen Leitungskatheter mit mehreren Einlass-Rückschlagventilen in Form von Folienventilen mit einer gemeinsamen ersten Folie und jeweils Gruppen von mehreren Löchern und zweiten Öffnungen,
Fig. 5 einen Leitungskatheter mit Auslass-Rückschlagventilen in Form von Folienventilen,
Fig. 6 einen Leitungskatheter mit mehreren außenliegenden Auslass-Rückschlagventilen in Form von Folienventilen in zwei verschiedenen Zuständen,
Fig. 7 einen Leitungskatheter mit alternativen Einlass-Folienventilen,
Fig. 8 eine zweite Öffnung mit dazu versetztem Loch und eine möglichen Befestigungslinie,
Fig. 9 einen Leitungskatheter mit einer Vielzahl von Einlass-Folienventilen und
Fig. 10 einen Leitungskatheter mit einem Auslass-Rückschlagventil in Form eines Folienventils im Bereich des Leitungsabschnitts abseits des Rahmens.

Übereinstimmende Teile tragen in den unterschiedlichen Figuren identische Bezugszeichen.

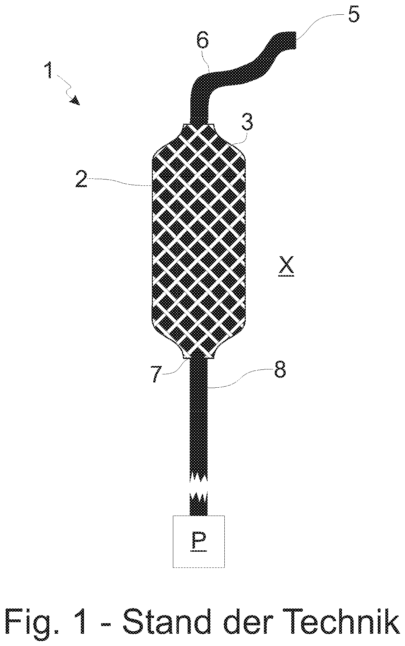

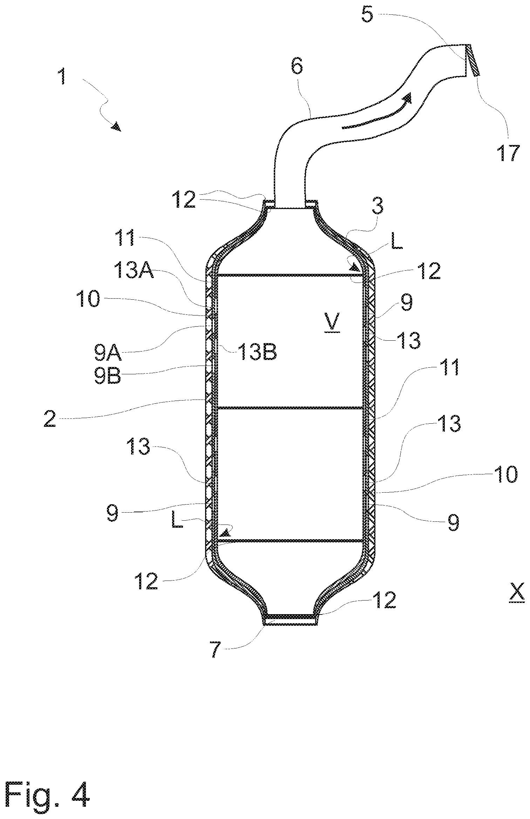

In **Fig. 1** ist ein bekannter Katheter 1 in schematischer Ansicht von außen dargestellt. Der Katheter 1 umfasst einen Metallkäfig als Rahmen 2. Im Inneren des Metallkäfigs ist eine Hülle 3 so angeordnet, dass diese einen Innenraum aufweist (nicht abgebildet). In dem Innenraum ist ein Ballon (nicht abgebildet) eines Ballonkatheters 4 angeordnet. Die Hülle 3 umfasst abseits des Rahmens 2 einen Schlauch als Leitung 6 für das zu transportierende Körperfluid, hier beispielsweise Blut, an dessen von dem Rahmen 2 entfernten Ende eine erste Öffnung 5 angeordnet ist. Die Hülle 3 weist mehrere zweite Öffnungen (nicht abgebildet) auf, an denen jeweils ein Rückschlagventil mit mechanischen Klappen (nicht abgebildet) als Einlassventil angeordnet ist. Zur Darstellung der zweiten Öffnungen und Rückschlagventile und ihrer Funktionsweise wird auf WO 2008/113785 A2, Fig. 4, verwiesen. Schließlich weist die Hülle 3 eine dritte Öffnung 7 auf, durch die der Ballonkatheter 4 in den Innenraum der Hülle 3 geführt ist. Aufgrund der Außenansicht ist der Ballon nicht erkennbar. Die Hülle 3 ist gegenüber dem lokalen Außenraum X fluiddicht verschlossen. Am von dem Innenraum abgewandten Ende der Fluidleitung 6 ist diese offen, so dass das Fluid vom Innenraum V durch die Leitung 6 transportiert werden und an deren Ende austreten kann. Die zweite Öffnung der Hülle 3 ist beispielsweise nur in Einlassrichtung fluiddurchlässig, in entgegengesetzter Strömungsrichtung ist sie durch das Rückschlagventil verschlossen. Die dritte Öffnung 7 der Hülle 3 ist durch die Leitung 8 des Ballonkatheters fluiddicht verschlossen. Diese Hilfsfluidleitung 8 ist mit einer extrakorporalen Pumpe P verbunden, die das Hilfsfluid alternierend in den Ballonkatheter pumpt und aus ihm abzieht. Der Ballonkatheter 4 dient damit als Antrieb für den Leitungskatheter 1 in Form einer Verdrängerpumpe, spezifischer einer Membranpumpe. Zur allgemeinen Funktionsweise wird auf WO 2008/113785 A2 verwiesen.

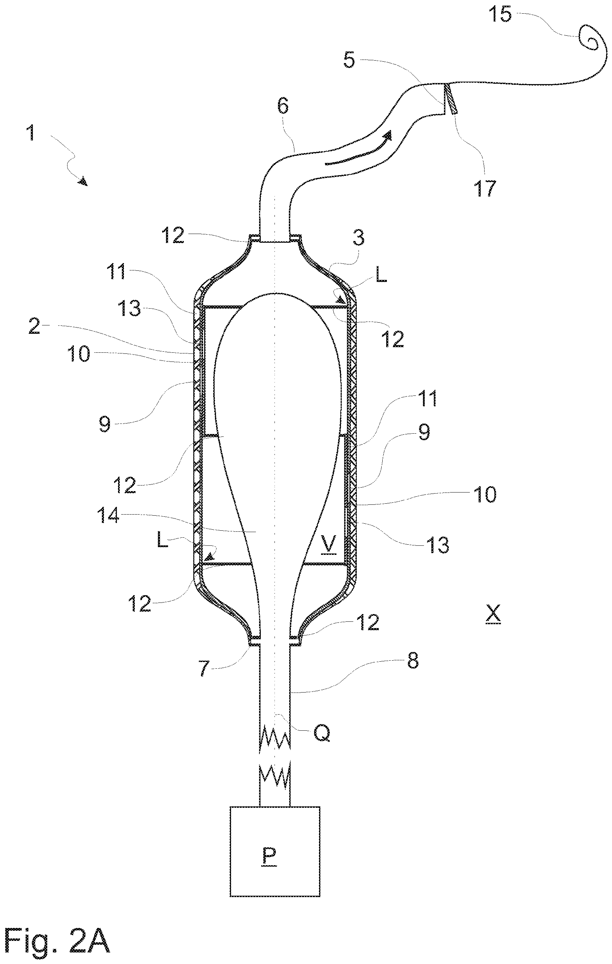

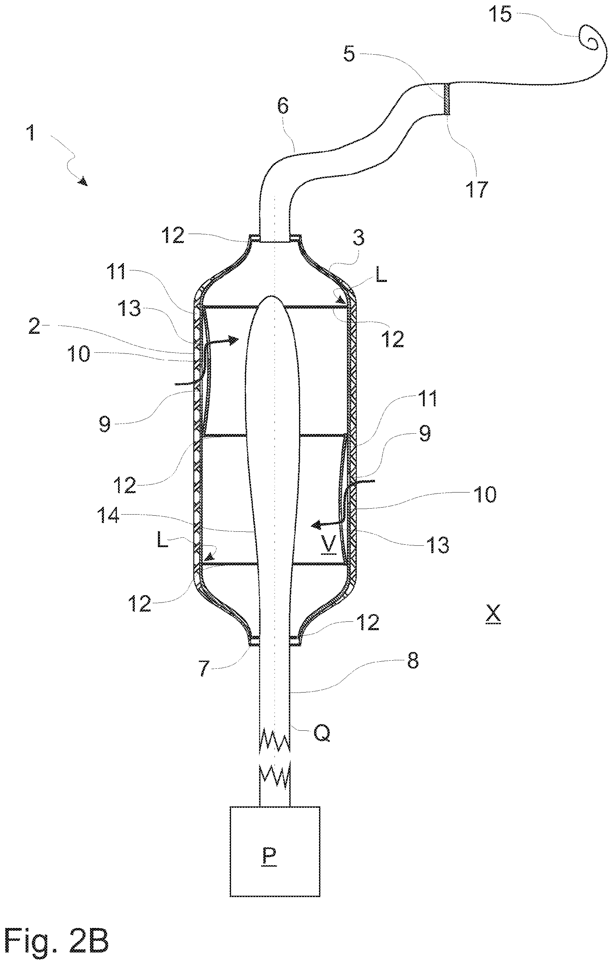

**Fig. 2** zeigt in einer schematischen Schnittdarstellung einen verbesserten Katheter 1, dessen Aufbau im Grundsatz dem in Fig. 1 gezeigten entspricht. Er kann vorzugsweise als Rechtsherzpumpkatheter verwendet werden. Die an den zweiten Öffnungen 9 der Hülle 3 angeordneten Rückschlagventile 10 sind als Folienventile ausgeführt. Zu diesem Zweck ist an jeder zweiten Öffnung 9 eine jeweilige Ventilfolie 11 mittels Ringen 12 entlang der durch den jeweiligen Außendurchmesser des betreffenden Rings gegebenen Linie L klemmend an der Hülle 3 befestigt. Zwischen den Ventilfolien 11 und dem Rahmen 2 ist die Hülle 3 in Form einer hier beispielhaft den gesamten Rahmen 2 auskleidenden Hüllfolie angeordnet. Sie ist durch die Ringe 12 ebenfalls klemmend am Rahmen 2 befestigt. In dem Innenraum V der Hülle 3 ist der Ballon 14 des Ballonkatheters 4 angeordnet, dessen Hilfsfluidleitung 8 durch die dritte Öffnung 7 zur Pumpe P nach außen geführt ist.

Durch die Klemmung gegen den Rahmen 2 ist die Hüllfolie 3 durch einen gegenüber dem Außenraum X geringeren statischen Druck im Innenraum V weniger weit von dem Rahmen 2 auslenkbar als die Ventilfolie 11, da sie durch den Rahmen 2 mit einer größeren inneren Zugspannung beaufschlagt ist als die Ventilfolie 11 und zudem eine kleinere Dehnbarkeit als die Ventilfolie 11 aufweist. Jede Ventilfolie 11 weist ein Loch 13 auf, das gegenüber der betreffenden zweiten Öffnung 9 der Hüllfolie 3 versetzt ist. Bei einem gegenüber dem Außenraum X geringeren statischen Druck im Innenraum V kann das zu transportierende Körperfluid vom Außenraum X durch die Öffnungen 9 und die von den Folien 11 und 3 gebildeten Rückschlagventile 10 in den Innenraum V strömen (Durchlassrichtung, dargestellt in Fig. 2B). Bei einem umgekehrten Druckverhältnis, beispielsweise während des Aufpumpens des Ballons 14 mittels des Hilfsfluides durch die Pumpe P, werden die Ventilfolien 11 gegen die Hülle 3 gepresst. Aufgrund des Versatzes zwischen den zweiten Öffnungen 9 und den Löchern 13 werden die zweiten Öffnungen 9 von den Ventilfolien 11 bedeckt und somit fluiddicht verschlossen (Sperrrichtung, dargestellt in Fig. 2A). Daher kann das zu transportierende Körperfluid nicht vom Innenraum V durch die Öffnung 9 in den Außenraum X strömen, sondern muss den Innenraum V durch die Leitung 6 verlassen.

Der Katheter 1 ist in dem Abschnitt, in dem der Rahmen 2 angeordnet ist, zwischen zwei Konfigurationen umstellbar, die sich hinsichtlich des Volumens des Innenraumes V und hinsichtlich des kleinsten äußeren Durchmessers des Rahmens 2 unterscheiden. Die Umstellbarkeit wird aufgrund der radiärsymmetrischen Struktur des Rahmens 2 um die Längsachse Q und seiner Zusammensetzung aus einer Formgedächtnislegierung, beispielsweise Nitinol, sowie der flexiblen Ausbildung der Rückschlagventile 10 und ihrer Anordnung in der Mantelfläche des näherungsweise zylindrischen Rahmens ermöglicht. In der ersten Konfiguration ist der Rahmen zusammengefaltet, so dass er an der dicksten Stelle einen Außendurchmesser von lediglich 20 Fr aufweist. In der zweiten Konfiguration ist er entfaltet, so dass der Ballon 14 aufgepumpt werden kann. Der Rahmen 2 kann beispielsweise aus einem an sich bekannten aufstellbaren Stent gebildet sein.

Die Folien 3 und 11 bestehen beispielsweise aus Polyurethan, können aber aus einem anderen Material, insbesondere einem anderen Polymer, bestehen. Sie sind beispielsweise 0,1 mm dick, so dass die Folienventile 10 jeweils eine Dicke von unter 1 mm aufweisen. Alle Öffnungen 5, 7, 9 der Hülle 3 sind beispielsweise kreisrund mit einer Öffnungsfläche von beispielsweise 5 mm², können aber jede andere Form und abweichende Größen aufweisen. Dasselbe gilt für die Löcher 13 der Ventilfolien 11. Die Öffnungen 5, 7, 9 und Löcher 13 sind beispielsweise durch Stanzen erzeugt, können aber auch mit einem Laser geschnitten oder anderweitig erzeugt sein.

An der ersten Öffnung 5, die am von dem Innenraum V abgewandten Ende der Fluidleitung 6 angeordnet ist, sind ein Rückschlagventil 17 als Auslassventil zur Verbesserung der Leitungseffizienz und eine elastische Spirale 15 zur besseren Fixierung des Leitungsendes in einer von der Gefäßwandung allseits beabstandeten Position angeordnet.

Jedes Folienventil 10 öffnet sich und lässt ein Fluid, beispielsweise Blut, durch die äußere Öffnung 9 fließen, wenn die Ventilfolie 11, die abseits der Ringe 12 nicht straff bezüglich des Rahmens 2, der aufgrund seiner relativen Steifigkeit den Innenraum V als Pumpkammer ausprägt, anliegt, durch den Antrieb (Sog beziehungsweise Unterdruckwirkung) des gerade entleerten inneren Ballons 14 (in der Systole bei einem Anwendungsbeispiel eines Pumpkatheters) wie in Fig. 2B etwas nach innen gezogen wird. Durch den kurzzeitig ausgebildeten Kanal zwischen der stets offenen äußeren Öffnung 9 und dem temporär nach innen gezogenen inneren Loch 13 kann also das Fluid durch das in der Regel pulsatil betriebene Folienventil 10 zeitlich gesteuert und gerichtet in die Pumpkammer V hineinfließen. Hingegen wird das Folienventil 10 in der Aufpumpphase des Ballons 14 (Diastole beim Anwendungsbeispiel eines Pumpkatheters) wie in Fig. 2A rhythmisch geschlossen, wenn der innere Katheter 4, beispielsweise ein IABP-Katheter, durch einen äußeren Gasstrom angetrieben stoßartig aufgepumpt wird und sich die Ventilfolie 11, bedingt durch den Druckanstieg in der Pumpkammer V, an die die Pumpkammer V auskleidende Hüllfolie 3 legt. In diesem Fall verschließen sich die versetzt angeordneten Öffnungen 9 und Löcher 13 beider Folien 3, 11 des Folienventils 10 und das Fluid, vorzugsweise Blut, das sich im Inneren der Pumpkammer V befindet, kann gerichtet in eine Vorzugsrichtung (im Fall eines Pumpkatheters in distaler Richtung entlang der Leitung 6) gepumpt werden. Die Transportrichtung ist durch einen Pfeil in der Leitung 6 beziehungsweise durch Pfeile in den Rückschlagventilen 10 dargestellt. Damit spielen die Folienventile 10, die in ihrer Anzahl und Form, wie sie auf der Mantelfläche eines intrakorporalen verwendbaren Katheters beliebig angeordnet und ausgeprägt sein können, eine entscheidende Rolle dafür, dass in einem Pumpkatheter für Flüssigkeiten, vorzugsweise Blut, eine Richtungsströmung ermöglicht wird.

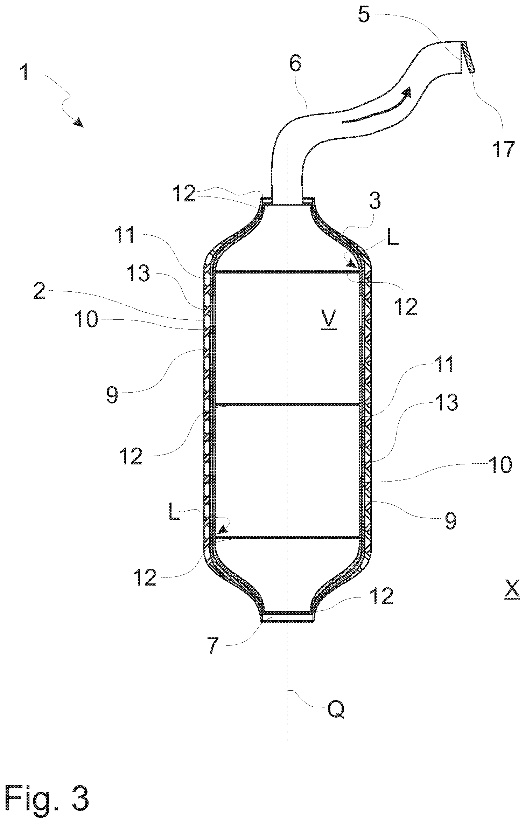

In **Fig. 3** ist in einer schematischen Schnittdarstellung ein gegenüber Fig. 2 modifizierter Leitungskatheter 1 abgebildet. Hier ist die Hülle 3, die wiederum aus einer Hüllfolie besteht, von einer einzelnen Ventilfolie 11 ausgekleidet, die mittels Ringen 12 an der Hüllfolie 3 befestigt ist. Die jeweiligen Segmente von Hüllfolie 3 und Ventilfolie 11 zwischen den einzelnen Ringen 12 unterscheiden sich in ihrer Auslenkbarkeit in Bezug auf den Rahmen 2 bei Unterdruck im Innenraum V. Die Segmente der Ventilfolie 11 können durch dieselbe Kraft weiter nach innen gezogen werden als die Segmente der Hüllfolie 3. Dies gelingt beispielsweise durch eine unterschiedliche Dehnbarkeit oder Spannung oder Geometrie der Folien 3 und 11.

Um die größere Auslenkbarkeit der ersten Folie 11 auf einem alternativen Weg zu realisieren, kann die erste Folie 11 beispielsweise an weniger Ringen 12 gegen den Rahmen 2 geklemmt sein als die Hülle 3.

Bei allen Ausführungsbeispielen können die dargestellten Ringe 12 beispielsweise aus Formgedächtniskeramik, Formgedächtnismetall oder aber als mechanische Verbindungsstellen durch Kleben, Klemmen oder Schweißen von Folie 11 und Hülle 3 ausgeführt sein. Die Ringe 12 können auch Teil des Rahmens 2 sein. Anstelle von separaten Ringen 12 können andere Geometrien vorgesehen sein, beispielsweise eine einzelne, durchgehende Spirale. Anstelle von separaten Ringen 12 können auch regelmäßig oder unregelmäßig verteilte beispielsweise punktförmige Verbindungsstellen (zum Beispiel eine Mehrzahl von Klebe- oder Schweißpunkten) vorgesehen sein.

**Fig. 4** zeigt in einer schematischen Schnittdarstellung eine gegenüber Fig. 3 modifizierte Ausführungsform. Jedes Rückschlagventil 10 umfasst dabei jeweils zwei zweite Öffnungen 9A und 9B in der Hülle 3 und dagegen versetzte zwei Löcher 13A und 13B in der Ventilfolie 11.

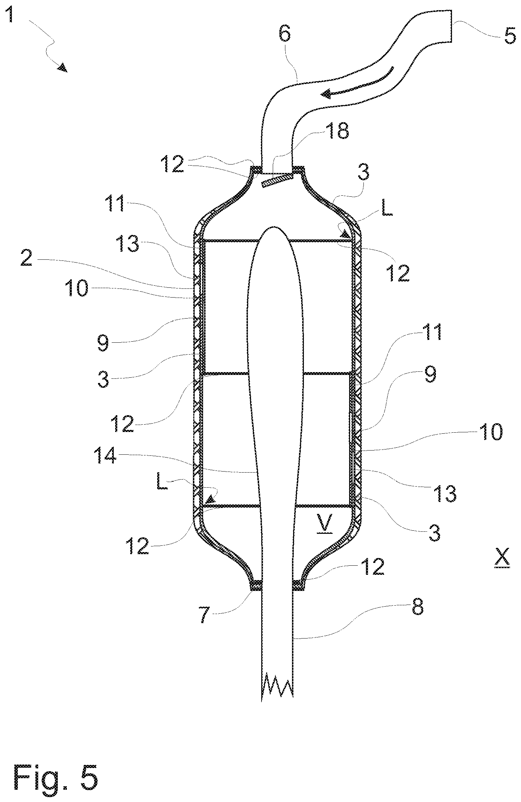

In **Fig. 5** ist ein gegenüber Fig. 2 modifizierter Katheter 1 wiederum in schematischer Schnittdarstellung gezeigt. Er kann vorzugsweise als Linksherzpumpkatheter verwendet werden. Hier sind die Rückschlagventile 10 als Auslass-Folienventil ausgebildet. Die Ventilfolien 11 sind zu diesem Zweck zwischen der Hülle 3 und dem Rahmen 2 angeordnet und wiederum beispielsweise punktuell verteilt oder entlang einer Befestigungslinie in der Art eines Ringes mit der Hülle 3 verbunden. Durch die weitere Auslenkbarkeit der Ventilfolie 11 kann diese bei Überdruck im Innenraum V in die Zwischenräume des den Rahmen 2 bildenden Nitinol-Stents ausweichen, so dass eine Strömung vom Innenraum V durch die zweiten Öffnungen 9 und die Löcher 13 in den Außenraum X ermöglicht wird. Bei Unterdruck im Innenraum V wird die Ventilfolie 11 gegen die Hülle 3 gezogen und verdeckt dadurch deren zweite Öffnungen 9, so dass die Rückschlagventile 10 sperren. Zur Unterstützung der Leitungseffizienz ist an dem am Rahmen 2 angeordneten Ende der Fluidleitung 6 ein Rückschlagventil 18 als Einlass-Ventil angeordnet.

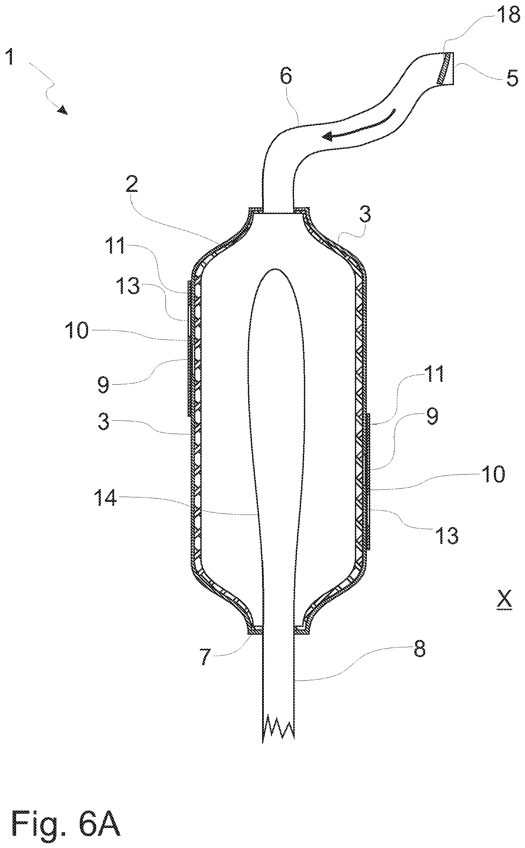

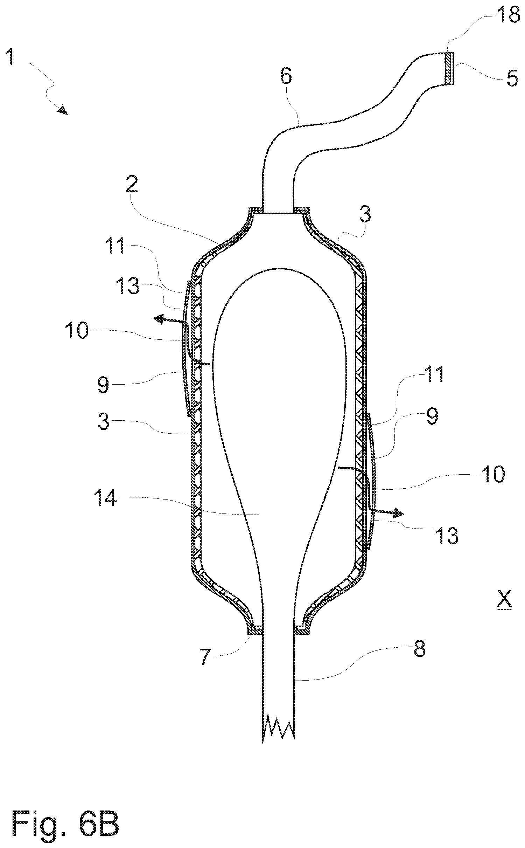

In **Fig. 6** ist ein gegenüber Fig. 2 modifizierter Katheter 1 wiederum in schematischer Schnittdarstellung gezeigt. Die Hülle 3 ist hier außerhalb des Rahmens 2 angeordnet und an ihm befestigt, beispielsweise geklebt oder geschweißt. Die erste Folie 11 ist zum Bereitstellen eines Auslassventils außen auf der Hülle 3 befestigt, beispielsweise durch ein Verbindungsverfahren wie Schweißen, Löten oder Kleben. Alternativ (nicht abgebildet) kann sie mittels Ringen durch Klemmen befestigt sein. Um in einer alternativen Ausführungsform (nicht abgebildet) die entgegengesetzte Transportrichtung zu realisieren, wäre die erste Folie 11 zum Bereitstellen eines Einlassventils auf der Innenseite der Hülle 3 angeordnet, beispielsweise innerhalb des Rahmens 2. Die erste Folie 11 wäre dann am Rahmen 2 und damit nur mittelbar an der Hülle 3 oder durch Zwischenräume des Rahmens 2 hindurch unmittelbar an der Folie 3 befestigt.

In Fig. 6B ist erkennbar, wie das Fluid beim Füllen des Ballons 14 aus dem Innenraum V durch die Folien-Auslassventile 10 gedrückt wird, indem die erste Folie 11 durch das Fluid von der Hülle 3 abgehoben wird. In Fig. 6A ist dargestellt, wie beim Leeren des Ballons 14 Fluid durch die Leitung 6 angesogen wird.

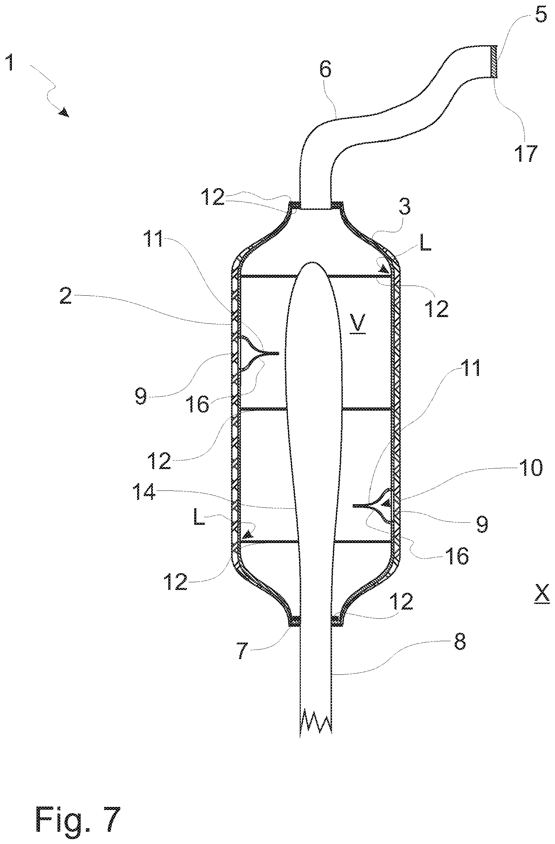

**Fig. 7** zeigt eine Ausführungsform mit einer alternativen Form von Folien-Rückschlagventilen 10. Sie weisen neben einer Ventilfolie 11 eine Zusatzfolie 16 auf, die teilweise flächig an der Ventilfolie 11 anliegt. Beide Folien sind rund um die betreffende zweite Öffnung 9 herum an der Hülle 3 befestigt, die beispielsweise ebenfalls aus einer Folie aus Polyurethan besteht. Bei Unterdruck im Innenraum V kann das Fluid die aneinanderliegenden Folien 11 und 16 auseinanderdrücken und so hindurchströmen. Bei Überdruck im Innenraum V hingegen werden die beiden Folien 11 und 16 gegeneinander gepresst und sind fluiddicht, so dass keine Strömung nach außen möglich ist.

Ein derartiges Folien-Einlassventil kann beispielsweise in der Ausführungsform gemäß Fig. 5 als Rückschlagventil 10 an dem am Rahmen 2 angeordneten Ende der Fluidleitung 6 eingesetzt werden. Dieselbe Bauform von Folien-Rückschlagventilen 10 kann auch als Auslassventil eingesetzt werden, insbesondere in anderen Ausführungsformen, beispielsweise gemäß Fig. 2, an der ersten Öffnung 5 am vom Rahmen 2 entfernten Ende der Fluidleitung 6.

In allen Ausführungsformen kann anstelle des Klemmens eine andere Art der Befestigung bereitgestellt sein.

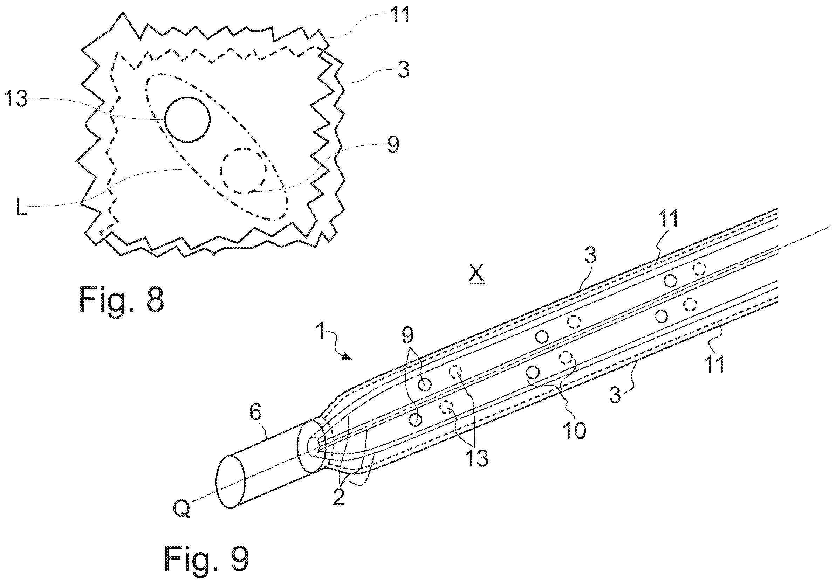

**Fig. 8** zeigt in einem schematischen Ausschnitt die Hülle 3 mit einer zweiten Öffnung 9 und die vor der Hülle 3 angeordnete Ventilfolie 11 mit einem gegenüber der zweiten Öffnung versetzten Loch 13. Rund um die Öffnung 9 und das Loch 13 gemeinsam ist die Ventilfolie 11 entlang der Linie L an der Hülle 3 befestigt, beispielsweise mit ihr verklebt oder verschweißt.

Sodann zeigt **Fig. 9** eine Ausführungsform eines erfindungsgemäßen Katheters 1 mit einer Vielzahl von Folien-Rückschlagventilen 10 in perspektivischer Darstellung. Der Rahmen 2 wird durch einen aufstellbaren lasergeschnittenen Nitinol-Stent gebildet, zwischen dessen einzelnen Längsstreben Zwischenräume ausgebildet sind. Die Hülle 3 wird durch einen flexiblen Folienschlauch gebildet. Der Rahmen 2 ist in den Hüllfolienschlauch 3 geschoben. Der Hüllschlauch 3 umspannt den Rahmen 2 zumindest in dessen aufgestellter Konfiguration straff, d.h. mit einer vorbestimmten Vorspannung. Der Rahmen dient somit der Aussteifung des durch die Hülle gebildeten Innenvolumens V, das in dem Ausführungsbeispiel der Fig. 9 eine Pumpkammer darstellt, in die der Ballon eines Ballonkatheters platzierbar ist. Die Hülle 3 weist mehrere lasergeschnittene oder gestanzte zweite Öffnungen 9 auf. Die Ventilfolie 11 ist ebenfalls ein einzelner Folienschlauch mit mehreren lasergeschnittenen oder gestanzten Löchern 13. Der Ventilfolienschlauch 11 ist zwischen dem Hüllfolienschlauch 3 und dem Rahmen 2 angeordnet. Die Folie des Ventilfolienschlauches 11 ist durch ihre im Vergleich zu der Hüllfolie 3 höhere Dehnbarkeit durch eine gegebene Kraft (entsprechend einem Unterdruck im Innenraum V) weiter in die Zwischenräume des Rahmens 2 in die Pumpkammer hinein auslenkbar als die Hüllfolie 3, so dass die Folien-Rückschlagventile 10 als Einlassventil arbeiten. Die zweiten Öffnungen 9 und die Löcher 13 sind in jeweils einer Spirale um die Längsachse Q der Pumpkammer angeordnet. Die Folienschläuche 3 und 11 sind gegeneinander so ausgerichtet, dass die Spirale der Löcher 13 abseits, also neben der Spirale der Öffnungen 9 liegt, so dass die Löcher 13 und die Öffnungen 9 so gegeneinander versetzt sind, dass sie einander nicht überlappen. Jeder Öffnung 9 ist somit ein Loch 13 zugeordnet, sodass beide zusammen jeweils ein Folienventil 10 ausbilden. Die beiden Folienschläuche 3, 11 sind an ihrem Ende entlang der Befestigungslinie L miteinander verklebt, indem entlang der Linie L ein zum Verkleben der Folien geeigneter Kleber ringförmig appliziert ist. Weiterhin sind die beiden Folienschläuche 3, 11 entlang der Längsachse Q in Abständen miteinander verbunden. Dazu sind zwei spiralförmige Klebebahnen (nicht dargestellt) ausgeführt, die beiderseits parallelversetzt zu den versetzten Spiralbahnen der Öffnungen 9 und der Löcher 13 verlaufen und somit eine Ventilkammer zwischen den beiden Folien abgrenzen, die sich entlang der Längsachse Q spiralförmig um die Pumpkammer windet. Die Ventilkammer kommuniziert über die Öffnungen 9 mit dem Außenraum X und über die Löcher 13 mit dem Innenraum V. Ihr Volumen ist im geschlossenen Zustand der Folienventile 10, d.h. wenn die Ventilfolie 11 gegen die Hülle 3 drückt, minimal. Im geöffneten Zustand, d.h. wenn die in Ihrer Beweglichkeit durch die Klebebahnen begrenzte Ventilfolie von der Hülle 3 abgehoben und in den Innenraum V hinein ausgelenkt ist, weist sie dagegen ein vorbestimmtes Volumen auf, dessen Größe unter anderem von der gegebenen Kraft abhängt, mit der die Folie des Ventilfolienschlauches 11 in die Pumpkammer hinein ausgelenkt wird.

In einer zu der Fig. 9 ähnlichen alternativen Ausführungsform (nicht gezeigt) mit ebenfalls einer Vielzahl von Folien-Rückschlagventilen 10 wird der Rahmen 2 wiederum durch einen aufstellbaren Nitinol-Stent gebildet. Die Hülle 3 und die Ventilfolie 11 werden wiederum jeweils durch einen flexiblen Folienschlauch gebildet. Der Rahmen 2 ist analog zu dem Ausführungsbeispiel der Fig. 9 in den Hüllschlauch 3 geschoben. Die Hülle umspannt den Rahmen 2 zumindest in dessen aufgestellter Konfiguration straff, so dass die Hülle eine durch den Rahmen 2 ausgesteifte Pumpkammer V ausbildet. Der Ventilfolienschlauch 11 ist wiederum zwischen dem Hüllfolienschlauch 3 und dem Rahmen 2 angeordnet. In Abweichung zu dem Ausführungsbeispiel der Fig. 9 sind die Öffnungen 9 und Löcher 13 in mehreren Gruppen zu jeweils sechs Öffnungen 9 und sechs Löchern 13 ringförmig um die Längsachse Q gegeneinander versetzt angeordnet, sodass sie einander nicht überlappen. Jeder Öffnung 9 ist ein Loch 13 zugeordnet, sodass beide zusammen jeweils ein Folienventil 10 ausbilden. In Abweichung zu dem Ausführungsbeispiel der Fig. 9 weist der Ventilfolienschlauch 11 einen größeren Durchmesser auf als die Hüllfolie. Durch diese Zulage prägt der in den Hüllfolienschlauch geschobene Ventilfolienschlauch 11 in Längsrichtung Q sechs Taschen aus. Entlang der sechs durch die Begrenzungslinien der Taschen gebildeten Befestigungslinien L ist die Ventilfolie 11 mit der Hülle 3 verklebt, sodass entlang der Längsachse Q zwischen den Befestigungslinien L sechs Ventilkammern ausgebildet sind. Die Ventilkammern sind in Bezug auf den Rahmen 2 so angeordnet, dass die Längsstreben des Rahmens 2 entlang der Befestigungslinien L verlaufen. Mit anderen Worten sind die Ventilkammern somit in den längs verlaufenden Zwischenräumen der Streben angeordnet, wobei jeder Ventilkammer jeweils eine zweite Öffnung 9 jeder Gruppe von zweiten Öffnungen 9 und jeweils ein Loch 13 jeder Gruppe von Löchern 13 zugeordnet ist. Jede Ventilkammer kommuniziert somit über die ihr zugeordneten Öffnungen 9 mit dem Außenraum X und über die ihr zugeordneten Löcher 13 mit dem Innenraum V. Im geschlossenen Zustand der Folienventile 10, d.h. wenn die Ventilfolie 11 gegen die Hülle 3 drückt, ist das Volumen der Ventilkammern minimal. Die Ventilfolie 11 liegt dann im wesentlichen, also abgesehen von den sich eventuell durch die Zulage ausbildenden Falten, flächig an der Hüllfolie 3 an, sodass die Folienventile 10 fluiddicht verschlossen sind. Im geöffneten Zustand, d.h. wenn die Ventilfolie 11 durch eine gegebene Kraft (entsprechend einem Unterdruck in der Pumpkammer V) von der Hülle 3 abgehoben und in den Innenraum V hinein ausgelenkt ist, weisen die Ventilkammern dagegen ein vorbestimmtes Volumen auf, dessen Größe unter anderem von der gegebenen Kraft und der Zulage abhängt.

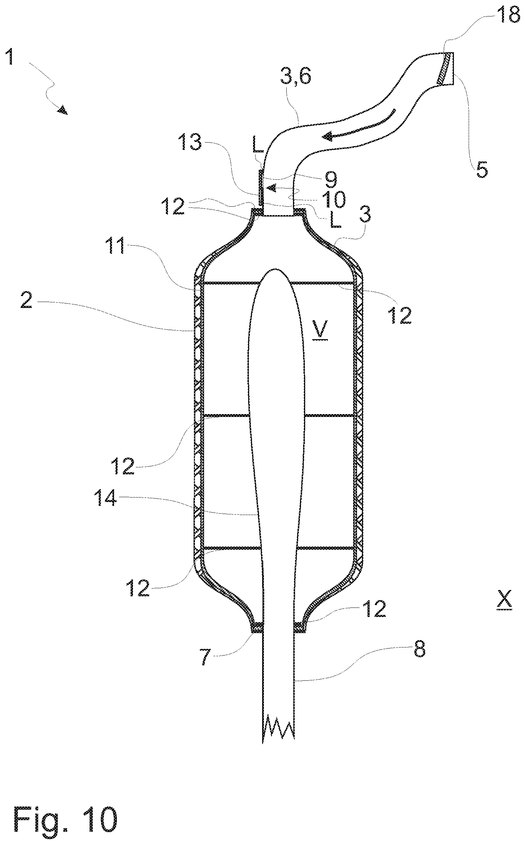

Schließlich zeigt **Fig. 10** einen gegenüber Fig. 2 modifizierten Katheter 1 wiederum in schematischer Schnittdarstellung. Hier ist die zweite Öffnung 9 in der Leitung 6 angeordnet. Der Pumpabschnitt des Innenraums V weist keine Öffnung zum Außenraum X auf. An der zweiten Öffnung 9 ist mittels einer ersten Folie 11 mit einem Loch 13 ein Folien-Auslassventil 10 realisiert. Am vom Rahmen 2 abgewandten Ende der Leitung 6 ist ein Einlass-Rückschlagventil 18 angeordnet. Erzeugt der Ballon 14 einen Überdruck Innenraum V, so wird das Fluid durch das Auslass-Folienventil 10 in den Außenraum X gedrückt. Zieht sich der Ballon 14 zusammen, entsteht ein Unterdruck im Innenraum V, wodurch Fluid durch das Einlassventil 18 in die Leitung und damit den Innenraum V gesogen wird. Im nächsten Überdruckzyklus wird das Fluid dann durch das Auslassventil 10 ausgestoßen und so längs der Leitung 6 transportiert.

In allen Ausführungsformen können mehrere zusätzliche Rückschlagventile 17 oder 18 an einer beliebigen Stelle der Leitung 6, insbesondere auf deren Längsachse, angeordnet sein. Alternativ oder zusätzlich kann das eine zusätzliche Ventil 17/18 oder die mehreren Ventile 17/18 abseits der Längsachse der Leitung 6 in einem Mantel der Leitung 6 angeordnet sein.

### Bezugszeichenliste

- 1: Katheter
- 2: Rahmen
- 3: Hülle
- 4: Ballonkatheter
- 5: Erste Öffnung
- 6: Leitung
- 7: Dritte Öffnung
- 8: Hilfsfluidleitung
- 9: Zweite Öffnung
- 10: Rückschlagventil
- 11: Ventilfolie
- 12: Ring
- 13: Loch
- 14: Ballon
- 15: Spirale
- 16: Zusatzfolie
- 17: Auslass-Rückschlagventil
- 18: Einlass-Rückschlagventil

- L: Linie
- V: Innenraum
- S: Segment
- P: Pumpe
- Q: Längsachse
- X: Außenraum

## Patentansprüche

1. Katheter (1) zum gerichteten Leiten einer Körperflüssigkeit, umfassend eine Hülle (3) mit einem Innenraum (V) und einen Rahmen (2), wobei
- die Hülle (3) mindestens drei Öffnungen (5, 7, 9) aufweist und zumindest in einem Bereich zwischen erster Öffnung (5) und zweiter Öffnung (9) als Leitung (6) für die Körperflüssigkeit ausgebildet ist,
- an der zweiten Öffnung (9) ein Rückschlagventil (10) angeordnet ist,
- die Hülle (3) einen Pumpkammerabschnitt aufweist,
- die zweite Öffnung (9) in dem Pumpkammerabschnitt der Hülle angeordnet ist,
**dadurch gekennzeichnet, dass**
- das Rückschlagventil (10) eine Ventilfolie (11) umfasst,
- die Ventilfolie innerhalb der Hülle (3) angeordnet ist, so dass das Rückschlagventil (10) als Einlassventil wirkt,
- die Ventilfolie (11) an der Hülle (3) zumindest teilweise befestigt ist, so dass die zweite Öffnung (9) durch die Folie (11) vollständig bedeckbar ist.

2. Katheter (1) nach Anspruch 1, wobei die Ventilfolie (11) entlang einer Befestigungslinie (L) mit der Hülle (3) verbunden ist und wobei die Ventilfolie (11) mindestens ein Loch (13) aufweist, das im vollständig bedeckten Zustand der zweiten Öffnung (9) abseits der zweiten Öffnung (9) angeordnet ist.

3. Katheter (1) nach Anspruch 2, wobei die zweite Öffnung (9) und das Loch (13) gemeinsam von der Befestigungslinie (L) umschlossen sind.

4. Katheter (1) nach Anspruch 2 oder 3, wobei das Loch (13) eine Fläche zwischen 5 mm² und 500 mm² aufweist oder wobei die Löcher (13) eines Rückschlagventils zusammen eine Gesamtfläche zwischen 5 mm² und 500 mm² aufweisen.

5. Katheter (1) nach einem der vorhergehenden Ansprüche, wobei die Hülle (3) zumindest abschnittsweise oder zumindest ihre äußere Einhüllende exakt oder im wesentlichen radiärsymmetrisch oder rotationssymmetrisch um eine Längsachse (Q) ist, und die zweite Öffnung (9) in einer Mantelfläche der Hülle (3) angeordnet ist..

6. Katheter (1) nach Anspruch 1, wobei die Hülle (3) zumindest in dem Pumpkammerabschnitt durch mindestens eine Hüllfolie gebildet ist.

7. Katheter (1) nach Anspruch 6, wobei die Hüllfolie weniger weit auslenkbar ist als die Ventilfolie (11) und/oder durch den Rahmen (2) mit einer größeren inneren Zugspannung beaufschlagt ist als die Ventilfolie (11).

8. Katheter (1) nach einem der vorhergehenden Ansprüche, wobei in dem Innenraum (V) ein Ballon (14) angeordnet ist und an den Ballon (14) eine Leitung (8) für ein Hilfsfluid zum Aufpumpen des Ballons (14) angeschlossen ist, wobei die Leitung (8) für das Hilfsfluid durch die dritte Öffnung (7) der Hülle (3) nach außen verläuft und mit einer Pumpe (P) für das Hilfsfluid verbindbar oder verbunden ist.

9. Katheter (1) nach einem der vorhergehenden Ansprüche, wobei in der Hülle (3) mehrere zweite Öffnungen (9) mit einem jeweiligen Rückschlagventil (10) angeordnet sind und jeweils eine Ventilfolie (11) oder ein jeweiliges durch Befestigungslinien definiertes Segment (S) der Ventilfolie (11) mit einem jeweiligen Loch (13) oder einer jeweiligen Gruppe von Löchern (13) zum Bedecken der betreffenden zweiten Öffnung (9) angeordnet ist.

10. Katheter (1) nach einem der vorhergehenden Ansprüche, wobei das Rückschlagventil (10) eine Gruppe mehrerer zweiter Öffnungen (9) umfasst, wobei von der Ventilfolie (11) diese Öffnungen (9) der betreffenden Gruppe vollständig bedeckbar sind.

11. Katheter (1) nach Anspruch 10, wobei die zu einem Rückschlagventil (10) gehörige Gruppe von zweiten Öffnungen (9) eine Gesamtfläche zwischen 5 mm² und 500 mm² aufweist und eine einzelne Öffnung der Gruppe von zweiten Öffnungen eine Fläche zwischen 0,25 mm² und 250 mm² aufweist.

12. Katheter (1) nach einem der vorhergehenden Ansprüche, wobei in der Leitung (6) für das Körperfluid ein weiteres Rückschlagventil (17, 18) angeordnet ist, das entgegengesetzt zu dem mindestens einen ersten Rückschlagventil (10) wirkt.

13. Katheter (1) nach einem der vorhergehenden Ansprüche, mit einer Zusammensetzung des Rahmens (2), die eine Formgedächtnislegierung, ein Formgedächtnispolymer oder eine Formgedächtniskeramik umfasst oder aus einer solchen besteht.

14. Katheter (1) nach einem der vorhergehenden Ansprüche, wobei die Ventilfolie (11) und/oder die Hüllfolie aus mindestens einem Polymer, bestehen, mit einer Foliendicke zwischen 0,01 mm und 0,2 mm.

15. Katheter (1) nach Anspruch 14, wobei das Polymer ein Polyurethan ist.

## Claims

1. Catheter (1) for the directional conductance of a bodily fluid, comprising a sheath (3) having an inner chamber (V) and a frame (2),
- the sheath (3) having at least three openings (5, 7, 9) and being formed as a conduit (6) for the bodily fluid at least in a region between a first opening (5) and a second opening (9),
- a non-return valve (10) being arranged at the second opening (9),
- the sheath (3) having a pump chamber portion,
- the second opening (9) being arranged in the pump chamber portion of the sheath,
**characterized in that**
- the non-return valve (10) comprises a valve film (11),
- the valve film is arranged inside the sheath (3) such that the non-return valve (10) acts as an inlet valve,
- the valve film (11) is fastened to the sheath (3) at least in part such that the second opening (9) can be fully covered by the film (11).

2. Catheter (1) according to claim 1, wherein the valve film (11) is connected to the sheath (3) along a fastening line (L) and wherein the valve film (11) has at least one hole (13), which, when the second opening (9) is in the fully covered state, is arranged remote from the second opening (9).

3. Catheter (1) according to claim 2, wherein the second opening (9) and the hole (13) are jointly encircled by the fastening line (L).

4. Catheter (1) according to either claim 2 or claim 3, wherein the hole (13) has an area of between 5 mm² and 500 mm² or wherein the holes (13) in a non-return valve together have a total area of between 5 mm² and 500 mm².

5. Catheter (1) according to any of the preceding claims, wherein the sheath (3), at least in portions or at least the outer envelope thereof, is exactly or substantially radially symmetrical or rotationally symmetrical about a longitudinal axis (Q), and the second opening (9) is arranged in a lateral surface of the sheath (3).

6. Catheter (1) according to claim 1, wherein the sheath (3) is formed by at least one enveloping film at least in the pump chamber portion.

7. Catheter (1) according to claim 6, wherein the enveloping film can be deflected to a lesser extent than the valve film (11) and/or the frame (2) applies a greater internal tensile stress on said enveloping film than on the valve film (11).

8. Catheter (1) according to any of the preceding claims, wherein a balloon (14) is arranged in the inner chamber (V) and a conduit (8) for an auxiliary fluid for pumping up the balloon (14) is connected to the balloon (14), wherein the conduit (8) for the auxiliary fluid runs outwards through the third opening (7) in the sheath (3) and is or can be connected to a pump (P) for the auxiliary fluid.

9. Catheter (1) according to any of the preceding claims, wherein a plurality of second openings (9) having respective non-return valves (10) are arranged in the sheath (3) and in each case a valve film (11), or a respective segment (S) of the valve film (11) defined by fastening lines, having a respective hole (13) or a respective group of holes (13) is arranged so as to cover the relevant second opening (9).

10. Catheter (1) according to any of the preceding claims, wherein the non-return valve (10) comprises a group of a plurality of second openings (9), wherein these openings (9) of the relevant group can be completely covered by the valve film (11).

11. Catheter (1) according to claim 10, wherein the group of second openings (9) that is associated with a non-return valve (10) has a total area of between 5 mm² and 500 mm², and an individual opening of the group of second openings has an area of between 0.25 mm² and 250 mm².

12. Catheter (1) according to any of the preceding claims, wherein a further non-return valve (17, 18) acting in the opposite direction to the at least one first non-return valve (10) is arranged in the conduit (6) for the body fluid.

13. Catheter (1) according to any of the preceding claims, having a composition of the frame (2) that comprises or consists of a shape-memory alloy, a shape-memory polymer or a shape-memory ceramic.

14. Catheter (1) according to any of the preceding claims, wherein the valve film (11) and/or the enveloping film consist(s) of at least one polymer, having a film thickness of between 0.01 mm and 0.2 mm.

15. Catheter (1) according to claim 14, wherein the polymer is a polyurethane.

## Revendications

1. Cathéter (1) destiné au transport dirigé d'un fluide corporel, comprenant une enveloppe (3) comportant un espace intérieur (V) et un cadre (2),
- l'enveloppe (3) présentant au moins trois ouvertures (5, 7, 9) et étant réalisé comme conduite (6) pour le fluide corporel, au moins dans une zone entre la première ouverture (5) et la deuxième ouverture (9),
- une soupape anti-retour (10) étant disposée au niveau de la deuxième ouverture (9),
- l'enveloppe (3) présentant une section de chambre de pompage,
- la deuxième ouverture (9) étant disposée dans la section de chambre de pompage de l'enveloppe, **caractérisé en ce que**
- la soupape anti-retour (10) comprend un film à soupape (11),
- le film à soupape est disposé à l'intérieur de l'enveloppe (3) de sorte que la soupape anti-retour (10) agit comme soupape d'entrée,
- le film à soupape (11) est au moins partiellement fixé à l'enveloppe (3) de sorte que la deuxième ouverture (9) peut être complètement recouverte par le film (11).

2. Cathéter (1) selon la revendication 1, dans lequel le film à soupape (11) est relié à l'enveloppe (3) le long d'une ligne de fixation (L) et le film à soupape (11) présentant au moins un trou (13) qui, à l'état complètement recouvert de la deuxième ouverture (9), est disposé à l'écart de la deuxième ouverture (9).

3. Cathéter (1) selon la revendication 2, dans lequel la deuxième ouverture (9) et le trou (13) sont entourés conjointement par la ligne de fixation (L).

4. Cathéter (1) selon la revendication 2 ou 3, dans lequel le trou (13) présente une surface comprise entre 5 mm² et 500 mm², ou dans lequel les trous (13) d'une soupape anti-retour présentent ensemble une surface totale comprise entre 5 mm² et 500 mm².

5. Cathéter (1) selon l'une des revendications précédentes, dans lequel l'enveloppe (3), au moins par sections ou au moins son extrémité d'enveloppe extérieure, est exactement ou sensiblement radialement symétrique ou symétrique en rotation autour d'un axe longitudinal (Q), et la deuxième ouverture (9) est disposée dans une surface externe de l'enveloppe (3).

6. Cathéter (1) selon la revendication 1, dans lequel l'enveloppe (3) est formée au moins dans la section de chambre de pompage par au moins un film d'enveloppement.

7. Cathéter (1) selon la revendication 6, dans lequel le film d'enveloppement est moins déformable que le film à soupape (11) et/ou est soumis à une contrainte de traction interne plus grande par le cadre (2) que le film à soupape (11).

8. Cathéter (1) selon l'une des revendications précédentes, dans lequel un ballonnet (14) est disposé dans l'espace intérieur (V) et une conduite (8) pour un fluide auxiliaire permettant de gonfler le ballonnet (14) est raccordée au ballonnet (14), la conduite (8) pour le fluide auxiliaire s'étendant vers l'extérieur à travers la troisième ouverture (7) de l'enveloppe (3), et pouvant être ou étant reliée à une pompe (P) pour le fluide auxiliaire.

9. Cathéter (1) selon l'une des revendications précédentes, dans lequel plusieurs deuxièmes ouvertures (9) comportant une soupape anti-retour respective (10) sont disposées dans l'enveloppe (3), et respectivement un film à soupape (11) ou un segment respectif (S) du film à soupape (11), lequel segment comporte un trou respectif (13) ou un groupe respectif de trous (13) et est défini par des lignes de fixation, étant disposé pour recouvrir la deuxième ouverture (9) correspondante.

10. Cathéter (1) selon l'une des revendications précédentes, dans lequel la soupape anti-retour (10) comprend un groupe de plusieurs deuxièmes ouvertures (9), lesdites ouvertures (9) du groupe en question pouvant être entièrement recouvertes par le film à soupape (11).

11. Cathéter (1) selon la revendication 10, dans lequel le groupe de deuxièmes ouvertures (9) appartenant à une soupape anti-retour (10) présente une surface totale comprise entre 5 mm² et 500 mm², et une ouverture individuelle du groupe de deuxièmes ouvertures présente une surface comprise entre 0,25 mm² et 250 mm².

12. Cathéter (1) selon l'une des revendications précédentes, dans lequel une autre soupape anti-retour (17, 18) est disposée dans la conduite (6) pour le fluide corporel, laquelle autre soupape anti-retour agit à l'encontre de l'au moins une première soupape anti-retour (10).

13. Cathéter (1) selon l'une des revendications précédentes, dont une composition du cadre (2) comprend un alliage à mémoire de forme, un polymère à mémoire de forme ou une céramique à mémoire de forme, ou est constituée de ceux-ci.

14. Cathéter (1) selon l'une des revendications précédentes, dans lequel le film à soupape (11) et/ou le film d'enveloppement sont constitués d'au moins un polymère comportant une épaisseur de film comprise entre 0,01 mm et 0,2 mm.

15. Cathéter (1) selon la revendication 14, dans lequel le polymère est un polyuréthane.
